# EUROPEAN PATENT SPECIFICATION

(11) **EP 1 282 601 B1**
(45) Date of publication and mention of the grant of the patent: **21.01.2004**
(21) Application number: 01929337.2
(22) Date of filing: 03.05.2001
(51) Int. Cl.: C07D 209/88, C07D 263/20, C07C 271/16, C07C 69/96

(54) **PROCESS AND INTERMEDIATES FOR THE PREPARATION OF 1-(9H-CARBAZOL-4-YLOXY)-3-[2-(2-METHOXY-PHENOXY)-ETHYLAMINO]-PROPAN-2-OL, CARVEDILOL OR ACID ADDITION SALT THEREOF**
VERFAHREN UND ZWISCHENVERBINDUNGEN ZUR HERSTELLUNG VON 1-(9H-CARBAZOL-4-YLOXY)-3-[2-(2-METHOXY-PHENOXY)-ETHYLAMINO]-PROPAN-2-OL, CARVEDIOL, ODER EIN SÄUREADDITIONSSALZ DAVON
PROCEDE ET INTERMEDIAIRES DESTINES A LA PREPARATION DE 1-(9H-CARBAZOL-4-YLOXY)-3- 2-(2-METHOXY-PHENOXY)-ETHYLAMINO]-PROPANE-2-OL, CARVEDILOL OU D'UN SEL D'ADDITION ACIDE DE CE COMPOSE

(30) Priority: 18.05.2000 DK 200000801
(43) Date of publication of application: 12.02.2003
(60) Divisional application: 03077684.3
(73) Proprietor: A/S GEA Farmaceutisk Fabrik, 2650 Hvidovre (DK)
(72) Inventor: PITTELKOW, Thomas, DK-2100 Copenhagen (DK); FISCHER, Erik, DK-2300 Copenhagen S (DK); TREPPENDAHL, Svend, Peter, DK-2830 Virum (DK)
(74) Representative: Simonsen, Christian Rosendal
(86) International application number: PCT/DK2001/000306
(87) International publication number: WO 2001/087837

(56) References cited:
- EP-A- 0 004 920
- EP-A- 0 918 055
- FR-A- 2 506 769

## Description

### FIELD OF INVENTION

The present invention relates to a new and advantageous process for the manufacture of 1-(9H-carbazol-4-yloxy)-3-[2-(2-methoxy-phenoxy)-ethylamino]-propan-2-ol of the formula (I).

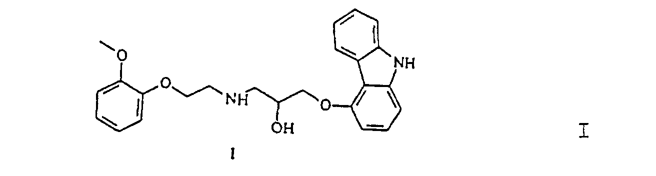

This compound is a valuable pharmaceutical substance which is used as a medicament having antihypertensive beta-adrenergic blocking and vasodilating activity. It is usually termed carvedilol and exists in at least two solid forms having differing physio-chemical properties. The conversion from one form to another is described in EP 0 893 440.

Further the invention relates to new intermediates useful in the preparation of carvedilol.

### BACKGROUND FOR THE INVENTION

Various routs of synthesis have been used or suggested for the preparation of carvedilol.

Thus EP 0 004 920 described reaction of 4-(oxiranylmethoxy)-9H-carbazol with 2-(2-methoxy-phenoxy)-ethylamine. 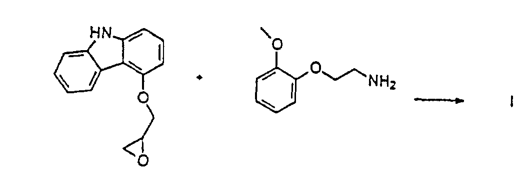 In this process also the bis compound (IV) is formed.

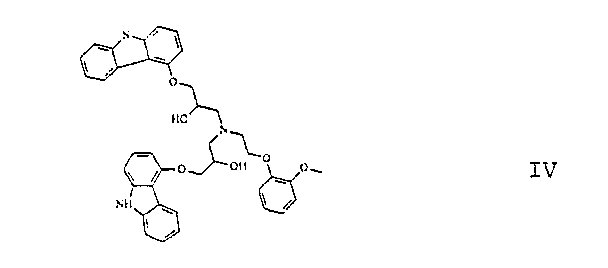

The starting compound 4-(oxiranylmethoxy)-9H-carbazol can be prepared by reaction of 4-hydroxycarbazol with an oxiranyl derivative, usually in a low yield. This means that a large amount of the expensive compound 4-hydroxycarbazol is lost during the reactions for which reason the process is less favourable in economical respect. In addition the synthesis makes use of an oxiranyl derivative which is not desired for environmental reasons.

Further EP 0 004 920 suggest three additional strategies for formation of carvedilol where 4-[3'-amino-2'-(hydroxy)-propoxy]-9H-carbazole is reacted with:
1. 2-[2'-methoxy-phenoxy]-ethylhalide or sulphonate,
2. 2-[2'-methoxy-phenoxy]-acetaldehyde followed by catalytic hydrogenation, or
3. 2-[2'-methoxy-phenoxy]-acetylchloride followed by reduction of the formed amide using a complex metal hydride.

Neither of these strategies are attractive from an industrial, economical point of view because the processes involve separation of the formed product from the starting materials using column chromatography and because the starting materials are relatively expensive.

In EP 0 127 099 the preparation of the optically active R and S enantiomers of carvedilol is described, using an R or an S enantiomer of the oxiran-derivative as starting material for the synthesis. Further it is described that the two enantiomers have differing biological activities. Thus the S enantiomer has a beta-blocking activity whereas both the R and the S enantiomer have a vasodilating activity.

EP 0 918 055 describes that the formation of the bis side product (IV) can be avoided by mono protecting the N atom in 2-(2'-(methoxy)-phenoxy)-ethylamine before reaction with 4-(oxiranylmethoxy)-9H-carbazol using a benzyl group as protection group. The formed product benzyl-carvedilol has to be debenzylated before the desired product carvedilol is formed, which debenzylation is done by hydrogenative debenzylation. Thus this procedure introduces two additional step in the synthesis, a benzylation and a debenzylation step.

In Maier, N.M. and Uray, G. (1996) J. Chromatogr. A, 740:11-19) the formation of 5-(9H-carbazol-4-yloxymethyl)-3-[2-(2-methoxy-phenoxy)-ethyl]-oxazolidin-2-one (IX) (the structure is shown below) is described by cyclisation of carvedilol with phosgene. The compound (IX) was used in a study of a particular stationary phase for use in chromatographic separations. Since according to this paper (IX) is made using carvedilol as starting material, the paper in no way inspires a person seeking new synthesis routes to carvedilol to consider (IX) as a starting material or an intermediate. The possibility of converting (IX) to carvedilol was not considered.

Thus there is a need for a new process for the synthesis of carvedilol not involving risks, hazards and high costs inherent in the above discussed prior art processes.

### BRIEF DESCRIPTION OF THE INVENTION

The present invention provides a new process for the manufacture of carvedilol which is economically and industrially feasible, which process do not make use of any hazardous oxiranyl derivatives.

The invention utilises the recognition that in a multi step synthesis it is advantageous to introduce the most expensive compound late in the synthesis in order to minimize the losses of the this compound, by minimizing the number of steps after its introduction.

Thus in one aspect the invention provides an process for the manufacture of 1-[9H-carbazol-4-yloxy]-3-[2-(2-methoxy-phenoxy)-ethylamino]-propan-2-ol, carvedilol, of the formula (I), or an acid addition salt thereof, 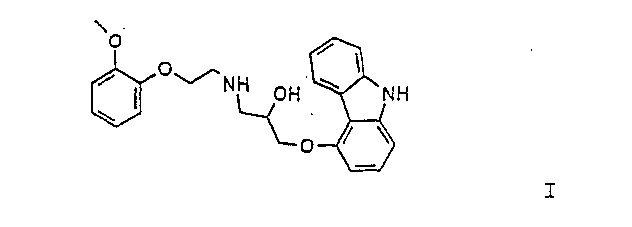 characterized in that a compound of the general formula (VII) 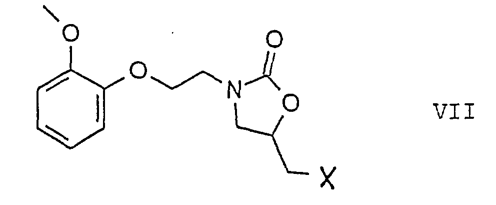 wherein X is a leaving group, where the leaving group X may be selected from, but not limited to: halogens (fluorine, chlorine, bromine and iodine), besylates, tosylate, mesylate, brosylates, nosylates, triflates, nonaflates or tresylates;
is alkylated with 4-hydroxycarbazol of the formula (VIII), 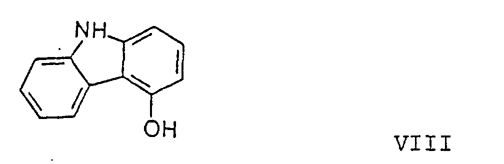 and the resulting compound 5-(9H-carbazol-4-yloxymethyl)-3-[2-(2-methoxy-phenoxy)-ethyl]-oxazolidin-2-one (IX) or an acid addition salt thereof 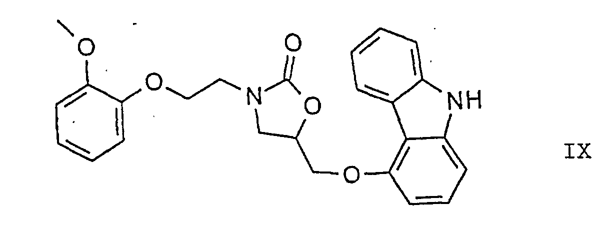 is hydrolysed.

This synthesis performs well in industrial scale with a yield of 50-60% (53% where X=chorine) based on the initial amount of the expensive compound 4-hydroxycarbazol.

Further the reaction proceeds without the use of any oxirane derivatives which is desirable for environmental reasons and for the safety of the staff.

From the Japanese patent application Sho 62-108870 3-alkyl-5-hydroxy-oxazolidin-2-one derivates are known as precursors for the preparation of β-blockers or α, β-blockers. However the compound having the general formula (VII) can not be produced by the disclosure in said patent application, nor can any hint for the synthesis of this compound be deduced from said application.

In one embodiment the alkylation of the compound having the general formula (VII) with 4-hydroxycarbazol and the hydrolysis of the resulting compound having the general formula (IX) to 1-(9H-carbazol-4-yloxy)-3-[2-(2-methoxy-phenoxy)-ethylamino]-propan-2-ol is performed without purification of the intermediate compound having the general formula (IX). Consequently these two reactions may be performed in one container without the need for handling of the reaction product of the first reaction and avoids the loss that inevitably occurs during handling of such a compound.

The compound having the general formula (VII) may be produced using several approaches.

In a preferred way of producing the compound of the general formula (VII) the oxazolidine ring of the compound is formed by cyclisation of a linear precursor having the general formula (VI) 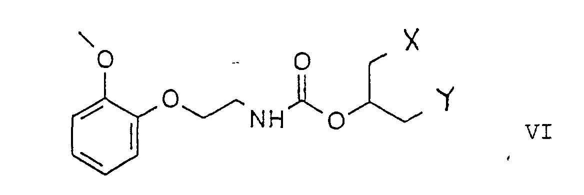 wherein X and Y each is a leaving group, where the leaving groups X and Y independently may be selected from but not limited to: halogens (fluorine, chlorine, bromine and iodine), besylates, tosylate, mesylate, brosylates, nosylates, triflates, nonaflates or tresylates.

### DETAILED DESCRIPTION OF THE INVENTION

The term leaving group is to be understood in the usual way as it will be appreciated by the person skilled in the art.

For the purpose of this invention the groups X, Y and Z are leaving groups and they may be the same or different, as the leaving groups are lost during the reactions to follow and non of them will be present in the final product, carvedilol. The term "leaving group" is to be understood in the usual meaning as it will be known to the person skilled in the art. Thus the groups X,Y and Z are selected so that the reactions in which compounds containing said groups participate can be performed at a suitable rate. The groups X and Y may independently be selected from: halogens (fluorine, chlorine, bromine and iodine), besylates, tosylate, mesylate, brosylates, nosylates, triflates or nonaflates. Z may be selected from: halogens (fluorine, chlorine, bromine and iodine). Chlorine is an example of a preferred group X,Y and Z. The person skilled in the art will appreciate that other leaving groups than the above mentioned, known within the art may be used for the present invention.

The compound (VI) is cyclisized for formation of the key compound of the general formula (VII) :

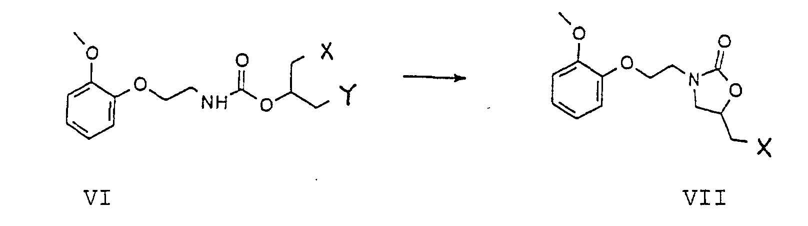

The reaction may be performed in a inert solvent in the presence of a base. The reaction is convenient performed by addition of a solution of compound of the general formula (VI)to a suspension or solution of the base in the absence of oxygen. An example of a suitable solvent for the reaction is tetrahydrofuran (THF) in the presence of sodium hydride as the base. This reaction may be performed at a temperature from 0°C to the reflux temperature of 67°C.

It will be appreciated by the person skilled in the art that the result of the above reaction may be two different compounds depending on whether X or Y have been eliminated during the ring closure. Consequently if X is different from Y the product of the reaction will be a mixture of two compounds each represented by the general formula (VII), one compound where the X-group in the general formula (VII) is the X-group from compound (VI), and one compound where the X-group is the Y-group from compound (VI).

Further it will be appreciated that the ratio of molecules containing X to molecules containing Y will depend on how well the groups X and Y perform as leaving groups. If Y is more easy to detach under the applied circumstances than X then the ratio will be high and vice versa.

As X and Y in the general formula (VI) are selected from the same definition of groups and because the groups will be lost during a subsequent step the notation the general formula (VII) in reactions where it participate will for simplicity only be mentioned with an X even though it strictly spoken may comprise more than one compound.

Formation of the compound of the general formula (VII) may be achieved in other ways. For example it may be produced by reaction of 1-(2-isocyanatoethoxy)-2-methoxy-benzene and epichlorohydrin. However this reaction has the disadvantage of using epichlorohydrin which is not desired because of the risk conferred by this chemical. Further, the formation of 1-(2-isocyanato-ethoxy)-2-methoxy-benzene involves the use of phosgene in the usual synthesis which is unacceptable from an environmental point of view.

Next the compound of the general formula (VII) is alkylated with 4-hydroxycarbazol for the formation of 5-(9H-carbazol-4-yloxymethyl)-3-[2-(2-methoxyphenoxy)-ethyl]-oxazolidin-2-one (IX):

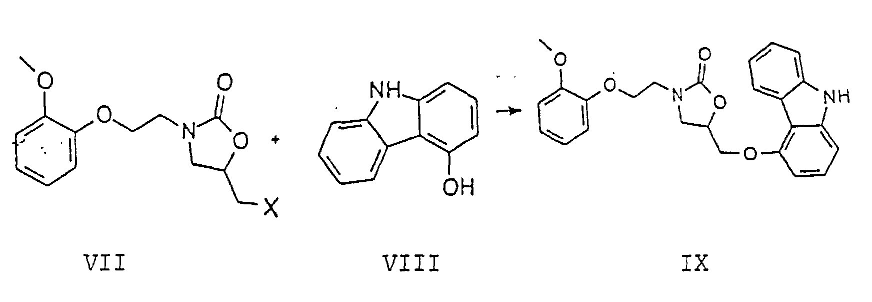

In principal the reaction may be performed in any ) solvent that is inert under the reaction conditions. However, surprisingly the selection of solvent greatly affects the reaction. In tetrahydrofuran the reaction hardly proceeds whereas the reaction performs well in alcohols having 1-6 carbon atoms.

A preferred solvent for the reaction is ethanol.

The reaction is most conveniently performed by refluxing a mixture of the reagents in a anhydrous alcohol such as absolute ethanol in the presence of a base such as potassium carbonate and a catalytic amount of potassium iodide.

The compound with the general formula (IX) may be purified in form of an acid addition salt, using methods well known for the person skilled in the art. The acid addition salt may in principle be an acid addition salt of any suitable acid. Examples of suitable acids for the formation of acid addition salts are: hydrochloric acid, hydrobromic acid, sulphuric acid, phosphoric acid, nitric acid, carbonic acid, acetic acid, butyric acid, succinic acid, malic acid, maleic acid, fumaric acid and citric acid.

As 4-hydroxycarbazol is very sensitive to oxygen and humidity in ambient air when warmed in solution the reaction is preferably performed under an inert atmosphere such as nitrogen or argon. Further it may be advantageous to add a small amount of an antioxidating agent such as potassium borohydride or sodium dithionite to improve the stability of 4-hydroxycarbazol under these conditions.

In the final step 5-(9H-carbazol-4-yloxymethyl)-3-[2-(2-methoxy-phenoxy) -ethyl]-oxazolidin-2-one (IX) or an acid addition salt thereof, is hydrolysed to 1-(9H-carbazol-4-yloxy)-3-[2-(2-methoxy-phenoxy)-ethylamino]-propan-2-ol, carvedilol, or an acid addition salt thereof:

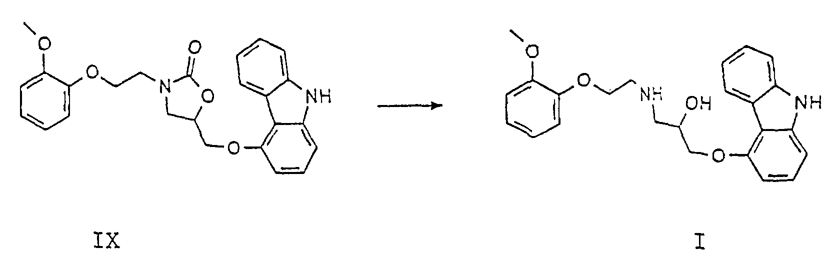

The hydrolysis may be performed by basic hydrolysis in a inert solvent in the presence of a base. A frequently used procedure for hydrolysis of a oxazolidine of this type involves refluxing of the compound in a mixture of ethanol and aqueous base. Such a procedure is also applicable for this particular case.

The reaction is conveniently performed in 96% ethanol using solid sodium hydroxide as base.

Alternatively 5-(9H-carbazol-4-yloxymethyl)-3-[2-(2-methoxy-phenoxy)-ethyl]-oxazolidin-2-one (IX) may be hydrolysed to the desired compound, carvedilol, under acidic conditions for formation of an acid addition salt. The acid addition salt may in principle be an acid addition salt of any suitable acid. The examples of suitable acids given above in connection with the formation of an acid salt of the compound with the general formula (IX) apply likewise for the formation of an acid addition of carvedilol.

The formation of an acid addition salt of the carvedilol may be convenient in some protocols for the purification of said compound. Once obtained, an acid addition salt of carvedilol may be converted to the free base, carvedilol, using methods well known within the area.

The two final steps, the alkylation of the compound having the general formula (VII) with 4-hydroxycarbazol and the subsequent hydrolysis of the compound having the general formula (IX) may be performed without purification of the intermediate product (IX). This allows the reaction to be performed in one container which would be advantageous because no purification or transfer of material is required which inevitably leads to loss and consumes time.

In order to perform these two final steps without intermediate purification a solvent should be selected in which both reactions performs well. An example of such a solvent is an alcohol having 1-6 carbon atoms, where ethanol is preferred.

Carvedilol contains one chiral carbon atom, the configuration of which is determined by the ring closure of the compound of the general formula (VI) to provide the compound of the general formula (VII) as shown above.

In one embodiment, the starting reagent is not a stereoisomer.

Racemic mixtures of the compound of the general formula (VII), (IX) or carvedilol may be resolved by methods known within the art.

In another embodiment the starting reagent is a stereoisomer, which means that X is different from Y, and the reagent is obtained in a steroeisomeric pure form i.e. either the R or the S enantiomer. The person skilled in the art will appreciate that as a result of the ring closure a mixture will be formed comprising a compound of the general formula (VII) in the R configuration containing exclusively either X or Y and a compound of the general formula (VII) in the S configuration containing exclusively the one of X and Y that is not contained in the R enantiomer. Thus the groups X and Y may serve as markers for the enantiomers.

In this embodiment X and Y may be selected so that one of the enantiomers is predominantly formed, by selection of X and Y such that one of these is a much better leaving group that the other. Alternatively X and Y may be selected to ease the separation of the R and S enantiomers of the compound of the general formula (VII) which enantiomers may be used to obtain carvedilol in the R and/or S configuration.

In a preferred embodiment X=Y=Z=chlorine and R=phenyl. The overall reaction scheme and the reactions for this embodiment is shown below. 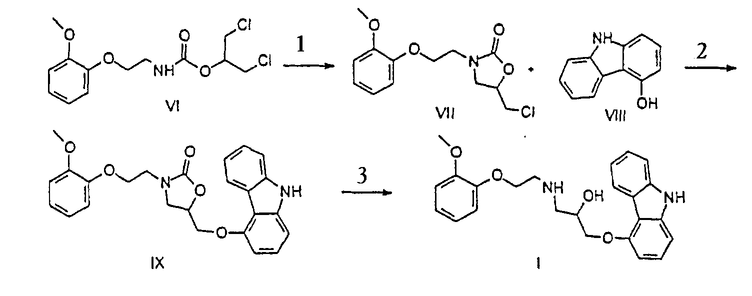
1. Cyclisation of [2-(2-methoxy-phenoxy)-ethyl]-carbamic acid 2-chloro-1-chloromethyl-ethyl ester (VI) with sodium hydride to 5-chloromethyl-3-[2-(2-methoxy-phenoxy)-ethyl]-oxazolidin-2-one (VII).
2. Alkylation of 5-chloromethyl-3-[2-(2-methoxyphenoxy)-ethyl]-oxazolidin-2-one (VII) with 4-hydroxycarbazol (VIII) to 5-(9H-carbazol-4-yloxymethyl)-3-[2-(2-methoxy-phenoxy)-ethyl]-oxazolidin-2-one (IX).
3. Alkaline hydrolyse of 5-(9H-carbazol-4-yloxymethyl)-3-[2-(2-methoxy-phenoxy)-ethyl]-oxazolidin-2-one (IX) to 1-(9H-carbazol-4-yloxy)-3-[2-(2-methoxy-phenoxy)-ethylamino]-propan-2-ol (I), carvedilol.

The invention is now further illustrated by way of examples. The examples should be regarded as illustratory and not construed as limiting in any way.

### EXAMPLES

### Example 1

### Production of 5-chloromethyl-3-[2-(2-methoxy-phenoxy)-ethyl]-oxazolidin-2-one (VII):

[2-(2-methoxy-phenoxy)-ethyl]-carbamic acid 2-chloro-1-chloromethyl-ethyl ester (VI) (46.8 g, 0.15 mol) in THF (100 ml) was added to sodium hydride (7.8 g, 0.20 mol, 60% in mineral oil) suspended in THF (100 ml) under nitrogen at a rate so that the temperature dit not exceed 67°C. The reaction mixture was stirred one hour at room temperature.
Next ligroin (bp=80-100°C)(50 ml) was added and water (100 ml) was carefully added to the mixture destroying excess of sodium hydride and dissolving the salts formed in the reaction.
The phases were separated and the organic phase was washed with water (50 ml).
The organic solution was concentrated under vacuum to afford the product.
Yield: 44.3 g (2.3 g mineral oil) 98%
An analytical sample was prepared by column chromatography on silica gel with ligroin (bp=80-100°C): ethyl acetate 1:1 as eluent.
NMR: ¹H (CDCl₃) 300 MHz δ = 7.00-6.87 (4H, m) , 4.74-4.66 (1H, m), 4.19 (2H, t, J=5Hz), 4.09-3.61 (4H, m), 3.85 (3H, s), 3.68 (2H, t, J=5Hz)

Elementary Analysis : C: 54.67 (54.65) H : 5.64 (5.64) N: 4.86 (4.90)
MS: 285 (M+, 2%), 162 (100%)
HPLC (nucleosil C18, CH₃CN/H₂O 1:1, eluent: phosphate / triethylamine buffer pH=3, flow 0.9 ml/min) RT=7.3 min

### Example 2

### Production of 5-(9H-carbazol-4-yloxymethyl)-3-[2-(2-methoxy-phenoxy)-ethyl]-oxazolidin-2-one (IX):

A mixture of 5-chloromethyl-3-[2-(2-methoxy-phenoxy)-ethyl]-oxazolidin-2-one (VII) (35.6 g, 0.12 mol), 4-hydroxycarbazol (24.7 g, 0.13 mol), potassium carbonate (34.6 g, 0.25 mol), potassium iodide (0.2 g) and sodium dithionite (0.2 g) in absolute ethanol (150 ml) was heated to reflux under nitrogen atmosphere for 24 hours.
Ligroin (bp=80-100°C)(25 ml) was added followed by water (50 ml). The reaction mixture was stirred for 24 hours, and the product removed by filtration and dried under vacuum.
Yield: 39.5 g (76%)
HPLC (nucleosil C18, CH₃CN/H₂O 1:1, eluent: phosphate / triethylamine buffer pH=3, flow 0.9 ml/min) RF=17.7 min

### Example 3

### Production of 1-(9H-carbazol-4-yloxy)-3-[2-(2-methoxyphenoxy)-ethylamino]-propan-2-ol, carvedilol (I):

5-(9H-carbazol-4-yloxymethyl)-3-[2-(2-methoxyphenoxy)-ethyl]-oxazolidin-2-one (IX) (10.0 g, 23.2 mmol) was refluxed in 96% ethanol (100 ml) containing sodium hydroxide (4.0 g, 0.1 mol) under a nitrogen atmosphere. After cooling to room temperature, a 1:1 mixture of toluene and THF (50 ml) followed by water (100 ml) were added to the reaction mixture. The phases were separated and the water phase extracted three times with a 1:1 mixture toluene and THF (50 ml).

The combined organic phase was dried, and concentrated under vacuum to afford the product as an oil.
The product was recrystallised from ethylacetate (25 ml).
Yield: 6.6 g (70%)
M.p: 114-116 °C
Elementary Analysis : C: 70.55 (70.92) H : 6.21 (6.45) N: 6.80 (6.89)
HPLC (nucleosil C18, CH₃CN/H₂O 1:1, eluent: phosphate / triethylamine buffer pH=3, flow 0.9 ml/min) RT=4.8 min

## Claims

1. A process for the manufacture of 1-(9H-carbazol-4-yloxy)-3-[2-(2-methoxy-phenoxy)-ethylamino]-propan-2-ol of the formula (I), or an acid addition salt thereof, 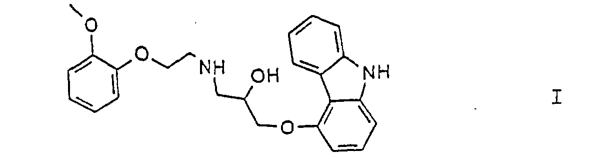 **characterized in that** a compound of the general formula (VII) 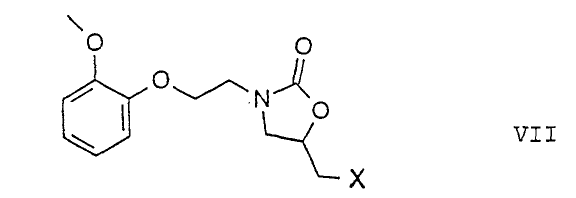 wherein X is a leaving group;
is alkylated with 4-hydroxycarbazol of the formula (VIII) , 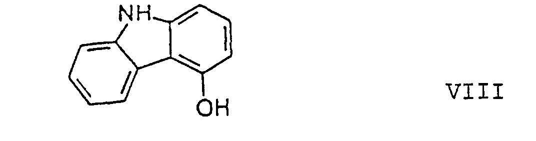 and the resulting compound 5-(9H-carbazol-4-yloxymethyl)-3-[2-(2-methoxy-phenoxy)-ethyl]-oxazolidin-2-one of the formula (IX), or an acid addition salt thereof 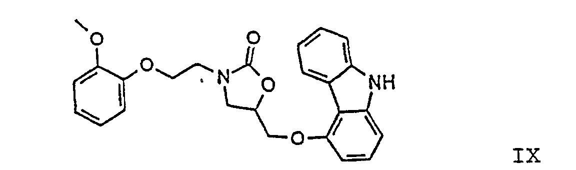 is hydrolysed.

2. A process according to claim 1, **characterized in that** X is selected among: fluorine, chlorine, bromine, iodine, besylates, tosylate, mesylate, brosylates, nosylates, triflates, nonaflates or tresylates.

3. A process according to claim 1 or 2, **characterized in that,** the alkylation of the compound having the general formula (VII) with 4-hydroxycarbazol, and the hydrolysis of the resulting compound having the general formula (IX) or an acid addition salt thereof, to provide 1-(9H-carbazol-4-yloxy)-3-[2-(2-methoxy-phenoxy)-ethylamino]-propan-2-ol or an acid addition salt thereof is performed in one container without isolation of the intermediate compound having the general formula (IX) or an acid addition salt thereof.

4. A process according to claim 1 to 3, **characterized in that** the hydrolysis of 5-(9H-carbazol-4-yloxymethyl)-3-[2-(2-methoxyphenoxy)-ethyl]-oxazolidin-2-one (IX) or an acid addition salt thereof is performed in basic medium.

5. A process according to claim 1-3, **characterized in that** the hydrolysis of 5-(9H-carbazol-4-yloxymethyl)-3-[2-(2-methoxyphenoxy)-ethyl]-oxazolidin-2-one (IX) or an acid addition salt thereof is performed in acidic medium.

6. A process according to claim 5, **characterized in that**, the addition salt of 1-(9H-carbasol-4-yloxy)-3-[2-(2-methoxy-phenoxy)-ethylamino]-propan-2-ol of the formula (I), is a addition salt of hydrochloric acid, hydrobromic acid, sulphuric acid, phosphoric acid, nitric acid, carbonic acid, acetic acid, butyric acid, succinic acid, malic acid, maleic acid, fumaric acid or citric acid.

7. A process according to any of claim 1 to 6, **characterized in that** the alkylation of compound VII with 4-hydroxycarbazol (VIII) is performed in a solvent which is an alcohol containing 1-6 carbon atoms.

8. A process according to claim 7, **characterized in that,** the solvent is ethanol.

9. A process according to any of the claims 1-8, **characterized in that** X=chlorine.

10. A compound having the general formula (VII) wherein X is a leaving group.

11. A compound according to claim 10, wherein X is selected from: fluorine, chlorine, bromine, iodine, besylates, tosylate, mesylate, brosylates, nosylates, triflates, nonaflates or tresylates.

12. The compound according to claim 10 or 11, **characterized in that** X=chlorine.

13. A process for the manufacture of a compound having the general formula (VII), wherein X is a leaving group; **characterized in that** compound of the general formula VI 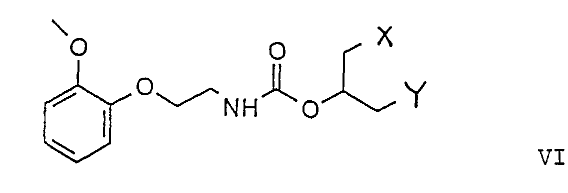 wherein X have the above signification, and Y is a leaving group;
is cyclisized.

14. A process according to claim 13; **characterized in that**, X and Y independently are selected from: fluorine, chlorine, bromine, iodine; besylates, tosylate, mesylate, brosylates, nosylates, triflates, nonaflates or tresylates.

15. A process according to claim 13-14, **characterized in that** X=Y=chlorine.

## Patentansprüche

1. Verfahren zur Herstellung von 1-(9H-Carbazol-4-yloxy)-3-[2-(2-methoxy-phenoxy)-ethylamino]-propan-2-ol der Formel (I), oder ein Säureadditionssalz davon, 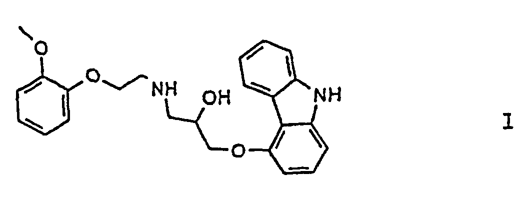 **dadurch gekennzeichnet, dass** eine Verbindung der allgemeinen Formel (VII) 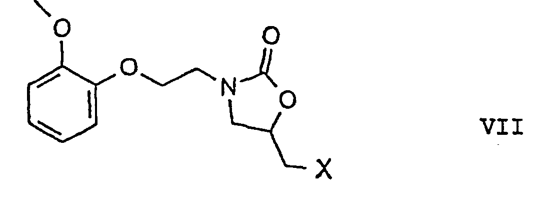 in welcher X eine Abgangsgruppe ist;
mit 4-Hydroxycarbazol der Formel (VIII) alkyliert wird, 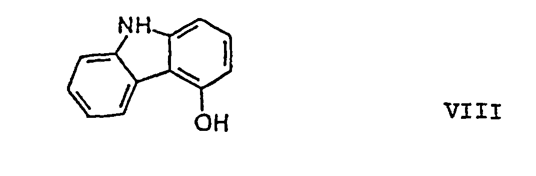 und die resultierende Verbindung 5-(9H-Carbazol-4-yloxymethyl)-3-[2-(2-methoxy-phenoxy)-ethyl]-oxazolidin-2-on der Formel (IX), oder ein Säureadditionssalz davon 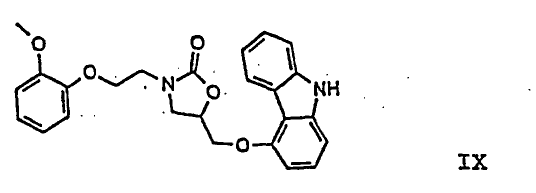 hydrolysiert wird.

2. Verfahren nach Anspruch 1, **dadurch gekennzeichnet, dass** X unter: Fluor, Chlor, Brom, Jod, Besylaten, Tosylat, Mesylat, Brosylaten, Nosylaten, Triflaten, Nonaflaten oder Tresylaten ausgewählt ist.

3. Verfahren nach Anspruch 1 oder 2, **dadurch gekennzeichnet, dass** die Alkylierung der Verbindung der allgemeinen Formel (VII) mit 4-Hydroxycarbazol, und die Hydrolyse der resultierenden Verbindung der allgemeinen Formel (IX) oder eines Säureadditionssalzes davon zum Erhalt von 1-(9H-Carbazol-4-yloxy)-3-[2-(2-methoxy-phenoxy)-ethylamino]-propan-2-ol oder eines Säureadditionssalzes davon in einem Behälter ohne Isolation der Zwischenverbindung der allgemeinen Formel (IX) oder eines Säureadditionssalzes davon durchgeführt wird.

4. Verfahren nach Anspruch 1-3, **dadurch gekennzeichnet, dass** die Hydrolyse von 5(9H-Carbazol-4-yloxymethyl)-3-[2-(2-methoxy-phenoxy)-ethyl]-oxazolidin-2-on (IX) oder eines Säureadditionssalzes davon in basischem Medium durchgeführt wird.

5. Verfahren nach Anspruch 1-3, **dadurch gekennzeichnet, dass** die Hydrolyse von 5-(9H-Carbazol-4-yloxymethyl)-3-[2-(2-methoxy-phenoxy)-ethyl]-oxazolidin-2-on (IX) oder eines Säureadditionssalzes davon in einem sauren Medium durchgeführt wird.

6. Verfahren nach Anspruch 5, dadurch **gekenneichnet, dass** das Additionssalz von 1-(9H-Carbazol-4-yloxy)-3-[2-(2-methoxy-phenoxy)-ethylamino]-propan-2-ol der Formel (I) ein Additionssalz von Hydrochlorsäure, Hydrobromsäure, Schwefelsäure, Phosphorsäure, Salpetersäure, Kohlensäure, Essigsäure, Buttersäure, Bernsteinsäure, Apfelsäure, Maleinsäure, Fumarsäure oder Zitronensäure ist.

7. Verfahren nach einem der Ansprüche 1-6, **dadurch gekennzeichnet, dass** die Alkylierung von Verbindung VII mit 4-Hydroxycarbazol (VIII) in einem Lösungsmittel, das ein Alkohol mit 1-6 Kohlenstoffatomen ist, durchgeführt wird.

8. Verfahren nach Anspruch 7, **dadurch gekennzeichnet, dass** das Lösungsmittel Ethanol ist.

9. Verfahren nach einem der Ansprüche 1-8, **dadurch gekennzeichnet, dass** X Chlor ist.

10. Verbindung der allgemeinen Formel (VII), in welcher x eine Abgangsgruppe ist.

11. Verbindung nach Anspruch 10, in welcher X unter: Fluor, Chlor, Brom, Jod, Besylaten, Tosylat, Mesylat, Brosylaten, Nosylaten, Triflaten, Nonaflaten oder Tresylaten ausgewählt ist.

12. Verbindung nach Anspruch 10 oder 11, **dadurch gekennzeichnet, dass** X Chlor ist.

13. Verfahren zur Herstellung einer Verbindung der allgemeinen Formel (VII), in welcher X eine Abgangsgruppe ist; **dadurch gekennzeichnet, dass** eine Verbindung der allgemeinen Formel VI 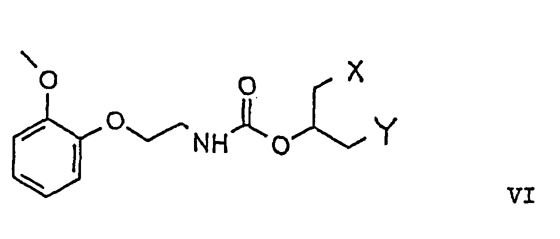 in welcher X die angegebene Bedeutung hat, und Y eine Abgangsgruppe ist;
zyklisiert wird.

14. Verfahren nach Anspruch 13, **dadurch gekennzeichnet, dass** X und Y unabhängig unter: Fluor, Chlor, Brom, Jod, Besylaten, Tosylat, Mesylat, Brosylaten, Nosylaten, Triflaten, Nonaflaten oder Tresylaten ausgewählt sind.

15. Verfahren nach Anspruch 13-14, **dadurch gekennzeichnet, dass** X=Y Chlor ist.

## Revendications

1. Procédé destiné à la préparation de 1-(9H-carbazol-4-yloxy)-3-[2-(2-methoxy-phenoxy)-éthylamino]-propane-2-ol de la formule (I), ou d'un sel additif d'acide de celui-ci, 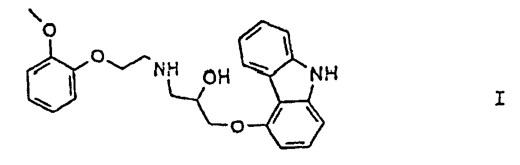 **caractérisé en ce qu'**un composé de la formule générale (VII) 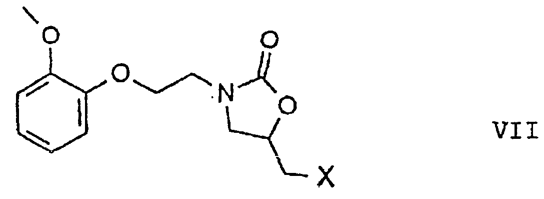 où X est un groupe de sortie ;
est alkylé avec 4-hydroxycarbazol de la formule (VIII), 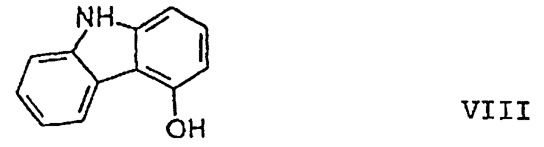 et le composé résultant 5-(9H-carbazol-4-yloxyméthyl)-3-[2-(2-methoxy-phenoxy)-éthyle]-oxazolidin-2-one de la formule (IX), ou d'un sel additif d'acide de celui-ci, 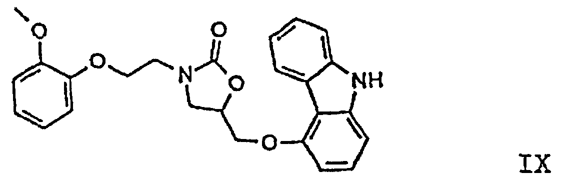 est hydrolysé.

2. Procédé selon la revendication 1, **ca-ractérisé en ce que** X est choisi parmi : fluorine, chlore, brome, iode, besylates, tosylate, mesylate, brosylates, nosylates, triflates, nonaflates ou tresylates.

3. Procédé selon la revendication 1 ou 2, **caractérisé en ce que** l'alkylation du composé ayant la formule générale (VII) avec 4-hydroxycarbazol, et l'hydrolyse du composé ayant la formule générale (IX) ou d'un sel additif acide de celui-ci, pour obtenir 1-(9H-carbazol-4-yloxy)-3-[2-(2-methoxy-phenoxy)-éthylamino]-propane-2-ol ou un sel additif d'acide de celui-ci, sont effectuées dans un conteneur sans isolation du composé intermédiaire ayant la formule générale (IX) ou d'un sel additif acide de celui-ci.

4. Procédé selon les revendications 1 à 3, **caractérisé en ce que** l'hydrolyse de 5-(9H-carbazol-4-yloxyméthyl)-3-[2-(2-methoxy-phenoxy)-éthyle]-oxazolidin-2-one (IX) ou d'un sel additif d'acide de celui-ci est effectuée dans un médium de base.

5. Procédé selon les revendications 1 à 3, **caractérisé en ce que** l'hydrolyse de 5-(9H-carbazol-4-yloxyméthyl)-3-[2-(2-methoxy-phenoxy)-éthyle]-oxazolidin-2-one (IX) ou d'un sel additif d'acide de celui-ci est effectuée dans un médium d'acide.

6. Procédé selon la revendication 5, **caractérisé en ce que** le sel additif de 1-(9H-carbazol-4-yloxy)-3-[2-(2-methoxy-phenoxy)-éthylamino]-propane-2-ol de la formule (I) est un sel additif d'acide hydrochlorique, d'acide hydrobromique, d'acide sulfurique, d'acide phosphorique, d'acide nitrique, d'acide carbonique, d'acide acétique, d'acide butyrique, d'acide succinique, d'acide malique, d'acide maléique, d'acide fumarique ou d'acide citrique.

7. Procédé selon l'une quelconque des revendications 1 à 6, **caractérisé en ce que** l'alkylation du composé VII avec 4-hydroxycarbazol (VIII) est effectuée dans un solvant qui est un alcool contenant 1-6 atomes carboniques.

8. Procédé selon la revendication 7, **caractérisé en ce que** le solvant est éthanol.

9. Procédé selon l'une quelconque des revendications 1 à 8, **caractérisé en ce que** X = chlore.

10. Composé ayant la formule générale (VII) où X est un groupe de sortie.

11. Composé selon la revendication 10, où X est choisi parmi : fluor, chlore, brome, iode, besylates, tosylate, mesylate, brosylates, nosylates, triflates, nonaflates ou tresylates.

12. Composé selon la revendication 10 ou 11, **caractérisé en ce que** X = chlore.

13. Procédé destiné à la préparation d'un composé ayant la formule générale (VII), où X est un groupe de sortie, **caractérisé en ce que** le composé de la formule générale VI 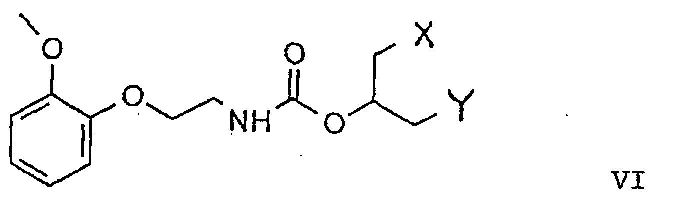 où X présente la signification plus haute, et Y est un groupe de sortie ;
est cyclisé.

14. Procédé selon la revendication 13, **caractérisé en ce que** X et Y indépendamment sont choisis parmi : fluor, chlore, brome, iode, besylates, tosylate, mesylate, brosylates, nosylates, triflates, nonaflates ou tresylates.

15. Procédé selon les revendications 13-14, **caractérisé en ce que** X = Y = chlore.
